# EUROPEAN PATENT APPLICATION

(11) **EP 4 260 911 A1**
(43) Date of publication of application: **18.10.2023**
(21) Application number: 21903484.0
(22) Date of filing: 10.12.2021
(51) Int. Cl.: A61Q 1/00, A61Q 19/00, A61K 8/34, A61K 8/35, A61K 8/39, A61K 8/81

(54) **COMPOSITION FOR FORMING COATING**

(30) Priority: 11.12.2020 JP 2020206184
(71) Applicant: Kao Corporation, Chuo-ku Tokyo 103-8210 (JP)
(72) Inventor: YOSHINO, Tatsuya, Tokyo 131-8501 (JP)
(74) Representative: Hoffmann Eitle
(86) International application number: PCT/JP2021/045450
(87) International publication number: WO 2022/124388

(57) **Abstract**

Provided is a composition for forming a coating film for forming a coating film formed of fibers directly on the skin by an electrostatic spray, the composition being capable of forming a coating film having excellent sebum resistance. The present invention relates to a composition for forming a coating film, comprising the following Components (a), (b), (c), and (d): (a) a polymer having a coating film forming ability, (b) one or more volatile substances selected from the group consisting of alcohols and ketones, (c) a plasticizer, (d) 3 mass% or more and 15 mass% or less of a non-volatile oil agent, other than Component (c), having a viscosity at 35°C of 50 mPa·s or more and 3,000 mPa·s or less.

## Description

### Field of the Invention

The present invention relates to a composition for forming a coating film on the skin.

### Background of the Invention

Various methods are known for forming a coating film by an electrostatic spray. For Example, Patent Literature 1 discloses a skin treatment method including electrostatically spraying a composition on the skin. The composition used in this method comprises a liquid insulating substance, an electrically conducting substance, a particulate powder substance, and a thickener. This composition is typically used as cosmetic products comprising a pigment and skin care compositions. Specifically, the composition is used as a makeup foundation. That is, the invention described in Patent Literature 1 mainly considers wearing a makeup on the skin by electrostatically spraying a makeup foundation for a cosmetic purpose.

Patent Literature 2 discloses a disposable cartridge to be used for an electrostatic spray apparatus of a cosmetic product. This electrostatic spray apparatus is a hand-held self-contained style. This electrostatic spray apparatus is used to spray a makeup foundation as in Patent Literature 1 described above.

Additionally, Patent Literature 3 discloses a method of producing a coating film on the skin by directly electrostatically spraying on the skin a composition comprising a solid oil at 20°C, in addition to a volatile substance and a polymer having a coating film forming ability.

Further, Patent Literature 4 discloses a composition for forming a coating film formed of fibers directly on the skin by an electrostatic spray, the composition comprising a plasticizer and a feel modifier, in addition to a volatile substance and a polymer having a coating film forming ability.

### Citation List

### Patent Literatures

[Patent Literature 1] JP-A-2006-104211
[Patent Literature 2] JP-A-2003-507165
[Patent Literature 3] JP-A-2018-177797
[Patent Literature 4] JP-A-2020-63226

### Summary of the Invention

The present invention relates to a composition for forming a coating film, containing the following Components (a), (b), (c), and (d):
(a) a polymer having a coating film forming ability
(b) one or more volatile substances selected from the group consisting of alcohols and ketones
(c) a plasticizer
(d) 3 mass% or more and 15 mass% or less of a non-volatile oil agent, other than Component (c), having a viscosity at 35°C of 50 mPa·s or more and 3,000 mPa·s or less.

### Brief Description of Drawings

[Figure 1] Figure 1 is a diagrammatic illustration showing a structure of the electrostatic spray apparatus preferably used in the present invention.
[Figure 2] Figure 2 is a schematic view showing a fashion for carrying out an electrostatic spray method using the electrostatic spray apparatus.

### Detailed Description of the Invention

When a coating film is formed on the skin by carrying out an electrostatic spray in accordance with the methods described in Patent Literatures 1 and 2, the adhesion between the skin and the coating film formed by the electrostatic spray is not sufficient and hence the coating film may be damaged or peeled due to an external force such as a friction. On the other hand, the coating films on the skin formed using the compositions of Patent Literatures 3 and 4 had excellent adhesion and also scratch resistance, but a phenomenon was proved to occur with the coating film formed on the skin being easily broken and dissolved with time.

Thus, the present invention relates to a composition capable of forming a coating film formed on the skin not only being excellent in adhesion and scratch resistance, but also protecting the skin without being broken with a long period of time.

Thus, the present inventor conducted various studies on the cause for the coating film formed on the skin to easily break with a long period of time, and found that the larger an amount of sebum secreted on the skin after formation of a coating film, the sooner the coating film breaks. As a result of continuation of further studies, it was found that when a non-volatile oil component having a viscosity within a certain range at 35°C is contained in a certain amount in addition to a polymer having a coating film forming ability, a volatile component, and a plasticizer, the composition with a strong resistance to sebum secreted on the skin surface on which a coating film is formed and capable of forming a coating film which is not easily broken can be obtained, leading to accomplishment of the present invention.

When a coating film formed of fibers is formed on the skin using the composition of the present invention, the obtained coating film not only has excellent skin compatibility, adhesion, and scratch resistance, but also has excellent sebum resistance. As a result, the coating film on the skin is not easily broken or dissolved with a long period of time, thereby being capable of protecting the skin for a long period of time.

The composition for forming a coating film of the present invention contains Components (a), (b), (c), and (d) described above. The coating film formed of fibers in the present invention means a coating film containing the fibers of Component (a) and may also be a coating film in which, for example, a liquid substance is present around the fibers other than the fibers.

The polymer having a coating film forming ability, Component (a), is a substance generally soluble in the volatile substance of Component (b). Soluble herein refers to a state in which, when Component (a) and Component (b) are mixed, Component (a) is in a dispersed state in Component (b) at 20°C and such a dispersed state is a visually homogenous state, and preferably a visually transparent or semitransparent state.

For the polymer having a coating film forming ability, a suitable polymer is used depending on the property of the volatile substance of Component (b). Specifically, the polymers having a coating film forming ability are roughly categorized into water-soluble polymers and water-insoluble polymers. The "water-soluble polymer" in the present Description refers to a polymer having a property that, under an environment of 1 atmosphere and 23°C, when 1 g of the polymer is weighed, subsequently immersed in 10 g of ion exchange water, and after having passed 24 hours, 0.5 g or more of the immersed polymer is dissolved in water. On the other hand, the "water-insoluble polymer" in the present Description refers to a polymer having a property that, under an environment of 1 atmosphere and 23°C, when 1 g of the polymer is weighed, subsequently immersed in 10 g of ion exchange water, and after having passed 24 hours, only less than 0.5 g of the immersed polymer is dissolved in water.

Examples of the water-soluble polymer having a coating film forming ability include mucopolysaccharides such as pullulan, hyaluronic acid, chondroitin sulfate, poly-γ-glutamic acid, modified cornstarch, β-glucan, glucooligosaccharide, heparin, and keratosulfate, natural polymers such as cellulose, pectin, xylan, lignin, glucomannan, galacturonic acid, psyllium seed gum, tamarind seed gum, gum arabic, gum tragacanth, water-soluble soybean polysaccharide, alginic acid, carrageenan, laminaran, agarose, fucoidan, methylcellulose, hydroxypropyl cellulose, and hydroxypropyl methylcellulose, synthetic polymers such as partially-saponified polyvinyl alcohol (when not used in combination with a crosslinking agent), low saponified polyvinyl alcohol, polyvinylpyrrolidone (PVP), polyethylene oxide, and sodium polyacrylate. These water-soluble polymers can be used singly or two or more can be used in combination. Of these water-soluble polymers, it is preferable to use pullulan, and synthetic polymers such as partially-saponified polyvinyl alcohol, low saponified polyvinyl alcohol, polyvinylpyrrolidone, and polyethylene oxide from a viewpoint of easy production of a coating film. When polyethylene oxide is used as the water-soluble polymer, a number average molecular weight thereof is preferably 50,000 or more and 3,000,000 or less, and more preferably 100,000 or more and 2,500,000 or less.

On the other hand, examples of the water-insoluble polymer having a coating film forming ability include completely-saponified polyvinyl alcohols insolubilizable after forming a coating film, partially-saponified polyvinyl alcohols crosslinkable after forming a coating film when used in combination with a crosslinking agent, oxazoline-modified silicones such as a poly(N-propanoylethyleneimine)graft-dimethylsiloxane/y-aminopropylmethylsiloxane copolymer, polyvinyl acetal diethylamino acetates, zein (the principal protein of maize), polyesters, polylactic acids (PLA), acrylic resins such as polyacrylonitrile resin, and polymethacrylic acid resin, and polystyrene resins, polyvinyl butyral resins, polyethylene terephthalate resins, polybutylene terephthalate resins, polyurethane resins, polyamide resins, polyimide resins, and polyamideimide resins. These water-insoluble polymers can be used singly or two or more can be used in combination.

Of these water-insoluble polymers, it is preferable to use completely-saponified polyvinyl alcohols insolubilizable after forming a coating film, partially-saponified polyvinyl alcohols crosslinkable after forming a coating film when used in combination with a crosslinking agent, polyvinyl butyral resins, polyurethane resins, and oxazoline-modified silicones such as a poly(N-propanoylethyleneimine)graft-dimethylsiloxane/y-aminopropylmethylsiloxane copolymer, polyvinyl acetal diethylamino acetates, and zein.

Component (a) is preferably a water-insoluble polymer having a coating film forming ability, and more preferably one or more selected from the group consisting of partially-saponified polyvinyl alcohols, low saponified polyvinyl alcohols, completely-saponified polyvinyl alcohols, polyvinyl butyral resins, polyurethane resins, polymethacrylic acid resins, oxazoline-modified silicones, polyvinyl acetal diethylamino acetates, and polylactic acids.

A content of Component (a) in the composition for forming a coating film of the present invention is preferably 1.0 mass% or more, more preferably 2.0 mass% or more, further preferably 4.0 mass% or more, furthermore preferably 6.0 mass% or more, and furthermore preferably 8.0 mass% or more. Additionally, it is preferably 35 mass% or less, more preferably 30 mass% or less, further preferably 25 mass% or less, and furthermore preferably 20 mass% or less. A content of Component (a) in the composition for forming a coating film is preferably 1.0 mass% or more and 30 mass% or less, more preferably 2.0 mass% or more and 25 mass% or less, further preferably 4.0 mass% or more and 20 mass% or less, and furthermore preferably 6.0 mass% or more and 20 mass% or less. Additionally, it is preferably 2.0 mass% or more and 35 mass% or less, more preferably 4.0 mass% or more and 30 mass% or less, further preferably 6.0 mass% or more and 30 mass% or less, furthermore preferably 6.0 mass% or more and 25 mass% or less, much preferably 8.0 mass% or more and 25 mass% or less, and much more preferably 8.0 mass% or more and 20 mass% or less. When Component (a) is comprised in this proportion in the composition for forming a coating film, an intended coating film can be efficiently formed and a coating film formed of fibers can be formed stably.

The volatile substance as Component (b) is a substance having volatility in a liquid state. Component (b) in the composition for forming a coating film is blended for the purpose of finally forming a dry coating film, wherein the composition for forming a coating film placed within the electric field is sufficiently charged, subsequently discharged toward the skin from a nozzle tip, a charge density of the composition for forming a coating film becomes excess as Component (b) evaporates, and Component (b) further evaporates while broken down by Coulomb repulsion. For this purpose, the volatile substance has a vapor pressure at 20°C of preferably 0.01 kPa or more and 106.66 kPa or less, more preferably 0.13 kPa or more and 66.66 kPa or less, further preferably 0.67 kPa or more and 40.00 kPa or less, furthermore preferably 1.33 kPa or more and 40.00 kPa or less, and much preferably 2.40 kPa or more and 40.00 kPa or less.

Of the volatile substances of Component (b), for example, monohydric chain fatty alcohols, monohydric cyclic fatty alcohols, and monohydric aromatic alcohols are preferably used as the alcohol. Examples of the monohydric chain fatty alcohol include straight chain or branched chain alcohols having 1 to 6 carbon atoms, examples of the monohydric cyclic fatty alcohol include cyclic fatty alcohols having 4 to 6 carbon atoms, and examples of the monohydric aromatic alcohol include benzyl alcohol and phenyl ethyl alcohol. Specific examples thereof include methanol, ethanol, isopropyl alcohol, n-propyl alcohol, n-butyl alcohol, 2-butyl alcohol, isobutyl alcohol, 2-methyl-2-propyl alcohol, n-pentanol, 2-pentanol, 3-pentanol, 2-methyl-1-butyl alcohol, 2-methyl-2-butyl alcohol, 3-methyl-1-butyl alcohol, 3-methyl-2-butyl alcohol, neopentyl alcohol, n-hexanol, 2-hexanol, 3-hexanol, 2-methyl-1-pentanol, 3-methyl-1-pentanol, 4-methyl-1-pentanol, 2-methyl-2-pentanol, 3-methyl-2-pentanol, 4-methyl-2-pentanol, 2-methyl-3-pentanol, 3-methyl-3-pentanol, 2,2-dimethyl-1-butanol, 2,3-dimethyl-1-butanol, 3,3-dimethyl-1-butanol, 2,3-dimethyl-2-butanol, 3,3-dimethyl-2-butanol, 2-ethyl-1-butanol, cyclobutanol, cyclopentanol, cyclohexanol, benzyl alcohol, and phenyl ethyl alcohol. These alcohols selected therefrom can be used singly or in combinations of two or more.

Of the volatile substances of Component (b), examples of the ketone include ketones having 2 alkyl groups having 1 to 4 carbon atoms such as acetone, methyl ethyl ketone, and methyl isobutyl ketone. These ketones can be used singly or two or more can be used in combination.

For the volatile substance of Component (b), one or more selected from the group consisting of the above alcohols and the above ketones can be used in combination.

The volatile substance of Component (b) is more preferably one or more selected from the group consisting of ethanol, isopropyl alcohol, and n-butyl alcohol, further preferably one or more selected from the group consisting of ethanol and isopropyl alcohol, and furthermore preferably ethanol.

A content of Component (b) in the composition for forming a coating film is preferably 45 mass% or more, more preferably 50 mass% or more, further preferably 55 mass% or more, and furthermore preferably 60 mass% or more. Additionally, it is preferably 98.8 mass% or less, more preferably 98 mass% or less, further preferably 97 mass% or less, furthermore preferably 96 mass% or less, much preferably 94 mass% or less, much more preferably 91 mass% or less, and much further preferably 88.5 mass% or less. A content of Component (b) in the composition for forming a coating film is preferably 50 mass% or more and 98.8 mass% or less, more preferably 50 mass% or more and 98 mass% or less, further preferably 55 mass% or more and 96 mass% or less, and furthermore preferably 60 mass% or more and 94 mass% or less. Additionally, it is preferably 45 mass% or more and 97 mass% or less, more preferably 50 mass% or more and 94 mass% or less, further preferably 50 mass% or more and 91 mass% or less, and furthermore preferably 50 mass% or more and 88.5 mass% or less. When Component (b) is comprised in this proportion in the composition for forming a coating film, an intended coating film can be efficiently formed and a coating film formed of fibers can be formed stably. Additionally, when Component (b) is comprised in this proportion in the composition for forming a coating film, Component (b) can be efficiently and sufficiently volatilized from the composition for forming a coating film when carrying out the electrostatic spray method.

A content mass ratio of Component (a) to Component (b), ((a)/(b)), in the composition for forming a coating film is, from a viewpoint of efficiently forming an intended coating film, a viewpoint of stably forming a coating film formed of fibers, and a viewpoint of efficiently and sufficiently volatilizing Component (b) from the composition for forming a coating film when carrying out the electrostatic spray method, preferably 0.010 or more, more preferably 0.060 or more, further preferably 0.080 or more, furthermore preferably 0.10 or more, much preferably 0.11 or more, and much more preferably 0.12 or more. Additionally, it is preferably 0.60 or less, more preferably 0.45 or less, further preferably 0.35 or less, furthermore preferably 0.33 or less, much preferably 0.30 or less, much more preferably 0.25 or less, much further preferably 0.20 or less, and much further preferably 0.18 or less. Such a (a)/(b) is preferably 0.010 or more and 0.60 or less, more preferably 0.060 or more and 0.33 or less, further preferably 0.10 or more and 0.25 or less, furthermore preferably 0.11 or more and 0.20 or less, and much preferably 0.12 or more and 0.18 or less. Additionally, it is preferably 0.060 or more and 0.45 or less, more preferably 0.080 or more and 0.35 or less, further preferably 0.10 or more and 0.33 or less, furthermore preferably 0.11 or more and 0.30 or less, and much preferably 0.12 or more and 0.25 or less.

Component (c) and Component (d) are not particularly limited as long as they are generally used in the field of cosmetic products but, for example, one or more selected from the group consisting of polyols, polyoxyalkylene glycols, polyoxyalkylene alkyl ethers, ester oils, silicone oils, hydrocarbon oils, liquid fat/oil, solid fat/oil, higher alcohols, and nonionic surfactants can be used singly or two or more can be used in combination.

The plasticizer, Component (c), is a component which imparts flexibility to a coating film formed of fibers by an electrostatic spray thereby enhancing the adhesion of the coating film to the skin and the scratch resistance, also enhancing the followability of the coating film along with the motion of the skin, and enhancing the skin compatibility of the coating film.

The plasticizer as Component (c) is selected from those evaluated, based on the following evaluation criteria, as presenting a plasticity ability to a water-insoluble polymer having a coating film forming ability of Component (a).

### (Evaluation procedure)

(1) 8.8 g of an oil agent to be evaluated is added to a mighty vial No. 4 (manufactured by Maruemu Corporation).
(2) 1.2 g of a specific Component (a) a polymer having a coating film forming ability is added to the vial of (1), stirred using a spatula to thoroughly disperse the polymer in the oil agent, and then the mighty vial is covered with a lid. The polymer, when in a powder form, is used for the evaluation as it is. The polymer, when in a solution form, is caused to deposit by removing a solvent, cut to small pieces having 3 mm x 3 mm x 3 mm or less, and used for the evaluation. The polymer, when already in small pieces having 3 mm x 3 mm x 3 mm or less at the time of being deposited, is used for the evaluation as it is. The polymer, when in a film form, is cut to small pieces having 3 mm x 3 mm x 3 mm or less, and used for the evaluation.
(3) The vial of (2) was rotationally stirred for 1 week using a mix rotor (MVR-3R (manufactured by AS ONE Corporation)) at a rotation speed of 100 r/min. (room temperature: 25°C)
(4) The vial of (3) is allowed to stand at 25°C for 2 hours and a state is visually observed.

### (Evaluation criteria)

- Plasticity ability presented: the polymer is completely dissolved in the oil agent (clear single-phase solution), or the polymer and the oil agent are mixed well and form a gel. However, when a part of the oil agent is separated (the polymer + oil agent phase, and the oil agent phase), the gel refers to the polymer + oil agent phase. (Shape of the polymer before evaluation is not maintained.)
- No plasticity ability presented: the polymer settles after the vial is allowed to stand and is dispersed again when the vial is shaken. The polymer settles again when the vial is further allowed to stand. The polymer is not dissolved or gelatinized. (Shape of the polymer before evaluation is maintained.)

The plasticizer as Component (c) is preferably a compound easily interactable with functional groups such as the hydroxy group, the ester, and the acetal in the structure of the polymer having a coating film forming ability, and examples specifically include polyols, polyoxyalkylene glycols (polyethers), polyoxyalkylene alkyl ethers, specific ester oils, specific silicone oils, and nonionic (non-ionic) surfactants but, from a viewpoint of enhancing the scratch resistance and a feel of a coating film, the plasticizer preferably comprises a polyol, a polyoxyalkylene glycol, a glycerin monofatty acid ester, a glycerin difatty acid ester, a malic acid diester, an N-acyl amino acid ester, ethylhexyl methoxycinnamate, or an alkyl benzoate. These compounds selected therefrom can be used singly or in combinations of two or more.

The polyol includes dihydric to hexahydric polyols and examples include such as ethylene glycol, propylene glycol, 1,3-propanediol, glycerin, and 1,3-butylene glycol.

Examples of the polyoxyalkylene glycol include diethylene glycols, triethylene glycols, polyethylene glycols, dipropylene glycols, tripropylene glycols, polypropylene glycols, dibutylene glycols, tributylene glycols, polybutylene glycols, diglycerins, triglycerins, polyglycerins, polyoxyalkylene glycols (those comprising: as the constituent unit of the oxyalkylene, one or more alkylene oxides such as ethylene oxide, propylene oxide, and butylene oxide), and mixtures thereof, polyoxyethylene glycerins, polyoxypropylene glyceryl ethers, polyoxybutylene glyceryl ethers, polyoxyalkylene glyceryl ethers (those comprising: as the constituent unit of the oxyalkylene, one or more alkylene oxides such as ethylene oxide, propylene oxide, and butylene oxide) and mixtures thereof.

Examples of the polyoxyalkylene alkyl ether include polyoxyethylene lauryl ethers, polyoxyethylene oleyl ethers, polyoxypropylene lauryl ethers, polyoxypropylene oleyl ethers, polyoxybutylene lauryl ethers, polyoxybutylene oleyl ethers, polyoxyalkylene alkyl ethers (those comprising: as the constituent unit of the oxyalkylene, one or more alkylene oxides such as ethylene oxide, propylene oxide, and butylene oxide, and an alkyl ether having an alkyl group having a straight chain or branched chain of about 1 to 20 carbon atoms) polyoxyethylene methyl glucosides, polyoxypropylene methyl glucosides, polyoxyalkylene methyl glucosides (those comprising: as the constituent unit of the oxyalkylene, one or more alkylene oxides such as ethylene oxide, propylene oxide, and butylene oxide) and mixtures thereof.

Examples of the specific ester oil include glycerin monofatty acid esters such as glyceryl monostearate, glyceryl monoisostearate, glyceryl monooleate, and glyceryl monopalmitate, glycerin difatty acid esters such as glyceryl distearate, glyceryl diisostearate, glyceryl dioleate, and glyceryl dipalmitate, lactic acid esters such as cetyl lactate and myristyl lactate, triethyl citrate, malic acid diesters such as diisostearyl malate, amino acid esters such as amino acid ester-2, acyl amino acid esters such as isopropyl lauroyl sarcosinate, ultraviolet absorbers such as ethylhexyl methoxycinnamate, and alkyl benzoates.

Examples of the specific silicone oil include amodimethicone, aminoethylaminopropyl dimethicone, aminopropyl dimethicone, oxazoline-modified silicone, PEG-11 methyl ether dimethicone, PEG/PPG-20/22 butyl ether dimethicone, polyglyceryl-3 disiloxane dimethicone, polyglyceryl-3 disiloxane ethyl dimethicone, and lauryl polyglyceryl-3 disiloxane ethyl dimethicone.

Examples of the nonionic surfactant include polyethylene glycol monofatty acid esters such as polyoxyethylene glycol monolaurate, and polyoxyethylene glycol monostearate, polypropylene glycol monofatty acid esters such as polyoxypropylene glycol monolaurate, and polyoxypropylene glycol monostearate, polybutylene glycol monofatty acid esters such as polybutylene glycol monolaurate, and polyoxybutylene glycol monostearate, polyoxyalkylene glycol monofatty acid esters (those comprising: as the constituent unit of the oxyalkylene, one or more alkylene oxides such as ethylene oxide, propylene oxide, and butylene oxide), and mixtures thereof, polyoxyalkylene glycol difatty acid esters (those comprising: as the constituent unit of the oxyalkylene, one or more alkylene oxides such as ethylene oxide, propylene oxide, and butylene oxide, and 2 fatty acids may be same kind or different kinds), and mixtures thereof, fatty acid polyoxyethylene sorbitan, maltitol hydroxy aliphatic alkyl ether, alkylated polysaccharides, alkylglycosides, sucrose fatty acid esters, polyoxyethylene hydrogenated castor oil glyceryl, polyoxyethylene sorbit fatty acid esters, polyoxyethylene-polyoxypropylene block copolymers tetrapolyoxyethylene·tetrapolyoxypropylene-ethylenediamine condensates, polyoxyethylene-beeswax-lanolin derivatives, alkanolamides, polyoxyethylene-propylene glycol fatty acid esters, polyoxyethylene-alkylamines, polyoxyethylene-fatty acid amides, alkylethoxydimethylamine oxides, trioleyl phosphates, and polyoxyethylene fatty acid glyceryls. These plasticizers can be used singly or two or more can be used in combination.

A content of Component (c) in the composition for forming a coating film is preferably 0.10 mass% or more, more preferably 0.50 mass% or more, further preferably 1.0 mass% or more, and furthermore preferably 1.5 mass% or more. Additionally, it is preferably 30 mass% or less, more preferably 25 mass% or less, further preferably 20 mass% or less, and furthermore preferably 15 mass% or less. A content of Component (c) in the composition for forming a coating film is preferably 0.10 mass% or more and 30 mass% or less, more preferably 0.50 mass% or more and 25 mass% or less, further preferably 1.0 mass% or more and 20 mass% or less, and furthermore preferably 1.0 mass% or more and 15 mass% or less. Additionally, it is preferably 0.50 mass% or more and 30 mass% or less, more preferably 1.0 mass% or more and 25 mass% or less, further preferably 1.0 mass% or more and 20 mass% or less, and furthermore preferably 1.5 mass% or more and 15 mass% or less. When a content of Component (c) is within this range, flexibility is imparted to a coating film formed of fibers by an electrostatic spray, the adhesion of the coating film to the skin and scratch resistance are enhanced, the followability of the coating film along with the skin is enhanced, and the skin compatibility of the coating film is enhanced.

A content mass ratio of Component (a) to Component (c), ((a)/(c)), in the composition for forming a coating film is, from a viewpoint of the flexibility, the skin compatibility, the adhesion and the scratch resistance of the coating film formed of fibers by an electrostatic spray, preferably 0.033 or more, more preferably 0.10 or more, further preferably 0.20 or more, furthermore preferably 0.40 or more, much preferably 0.80 or more, much more preferably 1.0 or more, and much further preferably 1.5 or more. Additionally, it is preferably 300 or less, more preferably 60 or less, further preferably 30 or less, furthermore preferably 20 or less, much preferably 15 or less, much more preferably 10 or less, and much further preferably 8.0 or less. Such an (a)/(c) is preferably 0.033 or more and 300 or less, more preferably 0.10 or more and 60 or less, further preferably 0.20 or more and 30 or less, furthermore preferably 0.40 or more and 10 or less, and much preferably 1.0 or more and 10 or less. Additionally, it is preferably 0.80 or more and 20 or less, more preferably 1.0 or more and 15 or less, further preferably 1.0 or more and 10 or less, and furthermore preferably 1.5 or more and 8.0 or less.

Component (d) is a non-volatile oil agent, other than Component (c), having a viscosity at 35°C of 50 mPa·s or more and 3,000 mPa·s or less. The non-volatile oil agent herein refers to a non-volatile oil agent at 20°C. On the other hand, the non-volatile oil agent having a viscosity at 35°C of 50 mPa·s or more and 3,000 mPa·s or less in the present invention means a viscosity at 35°C of the whole mixture comprising one or more non-volatile oil agents is 50 mPa·s or more and 3,000 mPa·s or less. Thus, the viscosity at 35°C of one constituent non-volatile oil agent does not need to be within this range, and the viscosity at 35°C of two or more non-volatile oil agents as a mixture may be within this range. For example, when a non-volatile oil agent having a viscosity at 35°C of less than 50 mPa·s is mixed with a non-volatile oil agent having a viscosity at 35°C of more than 3,000 mPa·s, the mixture may have a viscosity at 35°C of 50 mPa·s or more and 3,000 mPa·s or less. Alternatively, a thickener is added to the non-volatile oil agent having a viscosity at 35°C of less than 50 mPa·s and adjusts the viscosity at 35°C to be 50 mPa·s or more and 3,000 mPa·s or less to use.

For the non-volatile oil agents used as Component (d), one or more selected from the group consisting of ester oils, silicone oils, hydrocarbon oils, liquid fat/oil, solid fat/oil, and higher alcohols, other than Component (c), can be used in combination. Combinations having a viscosity at 35°C of 50 mPa·s or more and 3,000 mPa·s or less when one or more of these non-volatile oil agents are contained are used. From a viewpoint of obtaining excellent sebum resistance of a coating film, the viscosity at 35°C is preferably 100 mPa·s or more, more preferably 150 mPa·s or more, further preferably 170 mPa·s or more, and furthermore preferably 200 mPa·s or more, and preferably 2,800 mPa·s or less, more preferably 2,500 mPa·s or less, further preferably 2,000 mPa·s or less, and furthermore preferably 1700 mPa·s or less. Specifically preferable viscosities at 35°C is 100 mPa·s or more and 2,800 mPa·s or less, more preferably 150 mPa·s or more and 2,500 mPa·s or less, further preferably 170 mPa·s or more and 2,000 mPa·s or less, and furthermore preferably 200 mPa·s or more and 1,700 mPa·s or less.

Examples of the ester oil include octanoic acid esters such as cetyl octanoate, lauric acid esters such as hexyl laurate, myristic acid esters such as octyldodecyl myristate, palmitic acid esters such as octyl palmitate, stearic acid esters such as isocetyl stearate, isostearic acid esters such as isopropyl isostearate, isopalmitic acid esters such as octyl isopalmitate, oleic acid esters such as isodecyl oleate, adipic acid diesters such as diisopropyl adipate, sebacic acid diesters such as diethyl sebacate, malic acid diesters such as diisostearyl malate, isononanoic acid esters such as isononyl isononanoate and isotridecyl isononanoate, ethyl hexanoic acid esters such as cetyl ethylhexanoate, fatty acid esters such as neopentyl glycol diethylhexanoate, glycerides such as pentaerythrityl tetraethylhexanoate, triisostearin, glyceryl diisostearate, triethylhexanoin, (phytosteryl/behenyl) dimer dilinoleate, (phytosteryl/isostearyl/cetyl/stearyl/isostearl/cetyl/ste aryl/behenyl) dimer dilinoleate, phytosteryl macadamiate, pentaerythrityl tetra(behenate/benzoate/ethylhexanoate), tripropylene glycol dipivalate, diisopropyl sebacate, isodecyl neopentanoate, hexyldecyl dimethyloctanoate, cetyl lactate, myristyl lactate, acetylated lanolin, cholesteryl 12-hydroxystearate, ethylene glycol di-2-ethyl hexanoate, dipentaerythritol fatty acid ester, N-alkyl glycol monoisostearate, neopentyl glycol dicaprate, diisostearyl malate, glycerin di-2-heptylundecanoate, trimethylolpropane tri-2-ethylhexanoate, trimethylolpropane triisostearate, glycerin trioctanoate, glycerin triisopalmitate, trimethylolpropane triisostearate, cetyl 2-ethylhexanoate, pentaerythritol tetra-2-ethylhexanoate, glycerin tri-2-ethylhexanoate, 2-ethylhexyl palmitate, glycerin trimyristate, glyceride tri-2-heptyl undecanoate, castor oil fatty acid methyl ester, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, di-2-hexyldecyl adipate, triethyl citrate, glycerin monofatty acid ester, glycerin difatty acid ester, glycerin trifatty acid ester, acyl amino acid diesters such as di(phytosteryl/octyldodecyl) lauroyl glutamate, ultraviolet absorbers such as ethylhexyl methoxycinnamate, and alkyl benzoates.

Of these ester oils, from a viewpoint of obtaining a coating film having excellent sebum resistance, it is more preferable to use one or more selected from the group consisting of glycerides such as pentaerythrityl tetraethylhexanoate, glyceryl diisostearate, triethylhexanoin, pentaerythrityl tetra(behenate/benzoate/ethylhexanoate), dipentaerythritol fatty acid ester, glycerin di-2-heptylundecanoate, glycerin trioctanoate, glycerin triisopalmitate, glycerin tri-2-ethylhexanoate, glycerin trimyristate, glyceride tri-2-heptyl undecanoate, acetoglyceride, glycerin monofatty acid ester, glycerin difatty acid ester, glycerin trifatty acid ester, and acyl amino acid diesters such as di(phytosteryl/octyldodecyl) lauroyl glutamate.

Examples of the hydrocarbon oil include liquid paraffins, squalane, squalene, paraffins, isoparaffins, ceresin, isohexadecane, isododecane, ozokerite, pristane, paraffin waxes, vaselines, and microcrystalline waxes.

Of these hydrocarbons, from a viewpoint of obtaining a coating film having excellent sebum resistance, it is more preferable to use one or more selected from the group consisting of liquid paraffins, isododecane, paraffin waxes, and vaselines.

Examples of the liquid fat/oil include linseed oil, camellia oil, macadamia nut oil, corn oil, mink oil, olive oil, avocado oil, sasanqua oil, castor oil, safflower oil, rapeseed oil, soybean oil, peanut oil, triglycerin, glycerin trioctanoate, glycerin triisopalmitate, apricot kernel oil, cinnamon oil, jojoba oil, grapeseed oil, sunflower oil, almond oil, sesame oil, wheat germ oil, rice germ oil, rice bran oil, cotton seed oil, tea oil, evening primrose oil, egg-yolk oil, beef foot oil, cod-liver oil, and pentaerythrit tetraoctanoate.

Examples of the solid fat/oil include cocoa butter, coconut oil, palm oil, palm kernel oil, beef tallow, suet, lard, horse fat, hydrogenated oil, hydrogenated castor oil, Japan wax, and shea butter.

Examples of the higher alcohol include saturated straight chain monohydric alcohols and unsaturated monohydric alcohols. Examples of the saturated straight chain monohydric alcohol include dodecanol (lauryl alcohol), tridodecanol, tetradodecanol (myristyl alcohol), pentadecanol, hexadecanol (cetyl alcohol), heptadecanol, octadecanol (stearyl alcohol), nonadecanol, icosanol (arachidyl alcohol), heneicosanol, docosanol (behenyl alcohol), tricosanol, tetracosanol (carnaubyl alcohol), pentacosanol, and hexacosanol (ceryl alcohol). Examples of the unsaturated monohydric alcohol include oleyl alcohol and elaidyl alcohol.

Examples of the silicone oil include linear silicones such as dimethylpolysiloxane (dimethicone), methylphenyl polysiloxane, diphenyl polysiloxane (diphenyl dimethicone), diphenylsiloxy phenyl trimethicone, and methylhydrogen polysiloxane, cyclic silicones such as cyclopentasiloxane methyl trimethicone, octamethylcyclotetrasiloxane, decamethylcyclopentasiloxane, and dodecamethylcyclohexasiloxane, silicone resins forming a three-dimensional network structure, and silicone rubbers.

A content of Component (d) in the composition for forming a coating film is, from a viewpoint of obtaining a coating film having excellent sebum resistance, preferably 3 mass% or more and 15 mass% or less. Further, it is preferably 3.5 mass% or more and 15 mass% or less, more preferably 4 mass% or more and 15 mass% or less, and further preferably 5 mass% or more and 15 mass% or less.

A content mass ratio of Component (c) to Component (d), ((c)/(d)), in the composition for forming a coating film is, from a viewpoint of the flexibility, the skin compatibility, the adhesion and the scratch resistance of the coating film formed of fibers by an electrostatic spray, and further from a viewpoint of obtaining excellent sebum resistance of a coating film, preferably 0.0033 or more, more preferably 0.0050 or more, further preferably 0.0066 or more, furthermore preferably 0.01 or more, much preferably 0.03 or more, much more preferably 0.05 or more, much further preferably 0.066 or more, much furthermore preferably 0.1 or more, and much furthermore preferably 0.2 or more. Additionally, it is preferably 30 or less, more preferably 25 or less, further preferably 20 or less, furthermore preferably 15 or less, much preferably 10 or less, much more preferably 7.0 or less, much further preferably 5 or less, and much furthermore preferably 3 or less. Such a (c)/(d) is preferably 0.0033 or more and 30 or less, more preferably 0.0050 or more and 25 or less, further preferably 0.0066 or more and 20 or less, and furthermore preferably 0.01 or more and 15 or less. Additionally, it is preferably 0.03 or more and 10 or less, more preferably 0.05 or more and 7.0 or less, further preferably 0.066 or more and 5 or less, furthermore preferably 0.1 or more and 5 or less, and much furthermore preferably 0.2 or more and 3 or less.

A content mass ratio of Component (a) to Component (d), ((a)/(d)), in the composition for forming a coating film is, from a viewpoint of obtaining excellent sebum resistance of a coating film formed of fibers by an electrostatic spray, preferably 0.0033 or more, more preferably 0.005 or more, further preferably 0.0066 or more, furthermore preferably 0.01 or more, much preferably 0.02 or more, much more preferably 0.03 or more, much more preferably 0.066 or more, much more preferably 0.1 or more, much more preferably 0.3 or more, much more preferably 0.5 or more, and much more preferably 0.8 or more. Additionally, it is preferably 30 or less, more preferably 25 or less, further preferably 20 or less, furthermore preferably 15 or less, much preferably 13 or less, much more preferably 10 or less, and much further preferably 8.0 or less. Such a (a)/(d) is preferably 0.0033 or more and 30 or less, more preferably 0.005 or more and 25 or less, further preferably 0.0066 or more and 20 or less, furthermore preferably 0.01 or more and 15 or less, and much preferably 0.02 or more and 13 or less. Additionally, it is preferably 0.03 or more and 10 or less, more preferably 0.066 or more and 10 or less, further preferably 0.10 or more and 8.0 or less, furthermore preferably 0.30 or more and 8.0 or less, furthermore preferably 0.50 or more and 8.0 or less, and furthermore preferably 0.80 or more and 8.0 or less.

The composition for forming a coating film may comprise, in addition to the above components, a conductivity controlling agent, an oil agent other than Components (c) and (d), a coloring pigment, an extender pigment, a dye, a fragrance, a repellent, an antioxidant, a stabilizer, a preservative, vitamins, and water. The conductivity controlling agent is, from a viewpoint of the conductivity enhancement, preferably an alkali metal salt or an ammonium salt, more preferably an ionic surfactant, and further preferably one or more selected from the group consisting of cationic surfactants and anionic surfactants.

A content of the conductivity controlling agent in the composition for forming a coating film is not limited as long as an amount achieves the conductivity of the composition within the above ranges but, from a viewpoint of stably forming a coating film and a viewpoint of preventing a conductivity from excessively increasing, preferably 0.01 mass% or more and 10 mass% or less, more preferably 0.05 mass% or more, and further preferably 0.1 mass% or more, and more preferably 8 mass% or less, further preferably 6 mass% or less, furthermore preferably 2.5 mass% or less, and furthermore preferably 2 mass% or less.

A viscosity of the composition for forming a coating film at 25°C is, from a viewpoint of stably forming a coating film formed of fibers on the skin and a viewpoint of spinning property when electrostatically spraying, drying of fibers, and thinning diameters of fibers, preferably 2 mPa·s or more, more preferably 5 mPa·s or more, further preferably 10 mPa·s or more, furthermore preferably 30 mPa·s or more, much preferably 50 mPa·s or more, and much more preferably 80 mPa·s or more. Additionally, it is preferably 3,000 mPa·s or less, more preferably 2,000 mPa·s or less, further preferably 1,500 mPa·s or less, furthermore preferably 1,000 mPa·s or less, much preferably 800 mPa·s or less, and much more preferably 500 mPa·s or less. A viscosity of the composition for forming a coating film ranges preferably 2 mPa·s or more and 3,000 mPa·s or less, more preferably 5 mPa·s or more and 2,000 mPa·s or less, further preferably 10 mPa·s or more and 1,500 mPa·s or less, furthermore preferably 30 mPa·s or more and 1,000 mPa·s or less, much preferably 50 mPa·s or more and 800 mPa·s or less, and much more preferably 80 mPa·s or more and 500 mPa·s or less. A viscosity of the composition for forming a coating film is measured using a B type viscometer at 25°C. For the B type viscometer, for example, a B type viscometer (TVB-10M) manufactured by TOKI SANGYO CO., LTD. Can be used. The measurement condition in such a case has a measurement temperature of 25°C. The measurement temperature herein refers to a temperature of the composition for forming a coating film. Type of a rotor and a rotation speed of the rotor are selected in accordance with specifications of a measurement apparatus to be used depending on a viscosity of the composition for forming a coating composition. For example, when the above B type viscometer (TVB-10M) manufactured by TOKI SANGYO CO., LTD. Is used, the measurement can be achieved using an M2 rotor at 6 rpm when a viscosity of the composition for a coating film is 2,500 mPa·s or more, an M2 rotor at 12 rpm when such a viscosity is 1,000 mPa·s or more and less than 2,500 mPa·s, an M2 rotor at 30 rpm when such a viscosity is 500 mPa·s or more and less than 1,000 mPa·s, an M2 rotor at 60 rpm when such a viscosity is 100 mPa·s or more and less than 500 mPa·s, and an M1 rotor at 60 rpm when such a viscosity is less than 100 mPa·s. Additionally, instructions for use of the above B type viscometer (TVB-10M) manufactured by TOKI SANGYO CO., LTD. also include measurement conditions other than the above measurement conditions and a viscosity can also be measured under other measurement conditions depending on a viscosity of the composition for forming a coating film.

For forming a coating film formed of fibers on the skin using the composition for forming a coating film of the present invention, there is a method of forming a coating film formed of fibers directly on the skin by an electrostatic spray, and a method of forming a coating film formed of fibers on a substrate by an electrostatic spray and applying the coating film onto the skin. Subsequently described is a method for forming a coating film formed of fibers directly on the skin by an electrostatic spray.

The composition for forming a coating film is sprayed directly to a site at which a coating film is intended to be formed on the human skin by the electrostatic spray method. The electrostatic spray method comprises a step for electrostatically spraying the composition for forming a coating film to the skin using an electrostatic spray apparatus. The electrostatic spray apparatus basically has a container for accommodating the above composition, a nozzle for discharging the composition, a feed apparatus for feeding the composition accommodated in the container to the nozzle, and a power supply for applying a voltage to the nozzle.

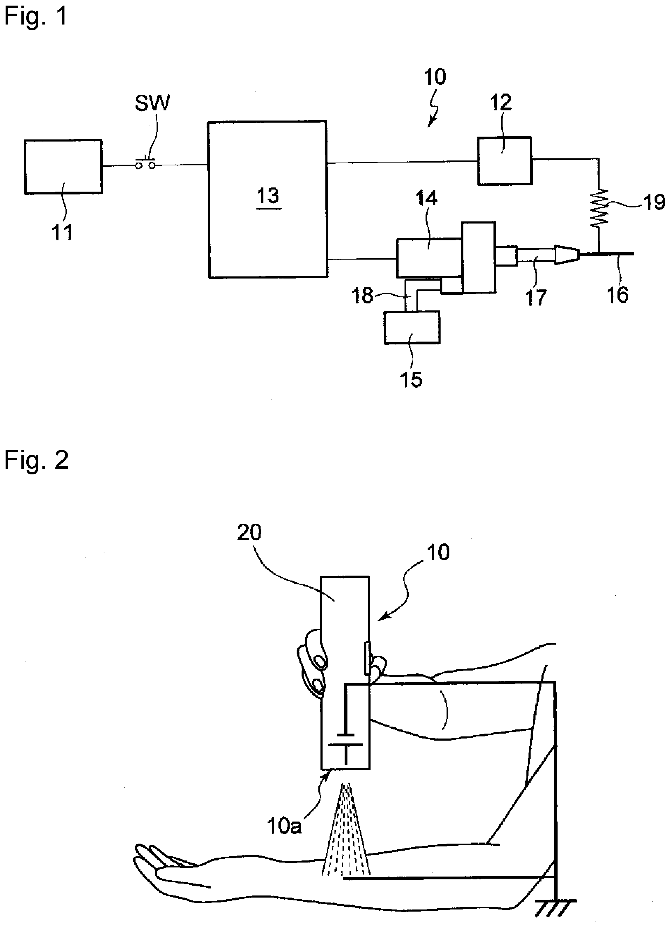

Figure 1 is a diagrammatic illustration showing a structure of the electrostatic spray apparatus preferably used in the present invention. An electrostatic spray apparatus 10 shown in the same figure is equipped with a low voltage power supply 11. The low voltage power supply 11 is for generating several to several tens of volt. The low voltage power supply 11 preferably consists of one or more batteries for the purpose of increasing the portability of the electrostatic spray apparatus 10. Additionally, batteries, when used as the low voltage power supply 11, can be easily replaceable as needed, hence advantageous. Instead of batteries, an AC adopter can also be used as the low voltage power supply 11.

The electrostatic spray apparatus 10 is also equipped with a high voltage power supply 12. The high voltage power supply 12 is connected to the low voltage power supply 11 and equipped with an electric circuit (not shown) to boost the voltage generated at the low voltage power supply 11 to a high voltage. A voltage boost electric circuit is generally made up of a transformer, a capacitor, and a semiconductor element.

The electrostatic spray apparatus 10 is further equipped with an auxiliary electric circuit 13. The auxiliary electric circuit 13 is interposed between the low voltage power supply 11 and the high voltage power supply 12 described above and functions to adjust a voltage of the low voltage power supply 11 thereby allowing the high voltage power supply 12 to operate stably. Further, the auxiliary electric circuit 13 has a function to control the rotation speed of a motor equipped by a micro gear pump 14 to be described later. Controlling the rotation speed of the motor controls a feed amount of the composition for forming a coating film to the micro gear pump 14 from a container 15 for the composition for forming a coating film. A switch SW is attached between the auxiliary electric circuit 13 and the low voltage power supply 11 and the electrostatic spray apparatus 10 can start/stop by switching ON-OFF the switch SW.

The electrostatic spray apparatus 10 is further equipped with a nozzle 16. The nozzle 16 consists of various conductors such as a metal to begin with and non-conductors such as plastic, rubber, and ceramic, and has a shape which can discharge the composition for forming a coating film from the tip thereof. Inside the nozzle 16, a microspace through which the composition for forming a coating film passes is formed longitudinally along with the nozzle 16. A cross-sectional size of this microspace is preferably 100 µm or more and 1,000 µm or less when expressed in the diameter.

The nozzle 16 communicates with the micro gear pump 14 via a pipeline 17. The pipeline 17 may be a conductor or a non-conductor. Additionally, the nozzle 16 is electrically connected to the high voltage power supply 12. This enables the application of a high voltage to the nozzle 16. In this case, the nozzle 16 and the high voltage power supply 12 are electrically connected via an electric current limiting resistor 19 to prevent an excess electric current from flowing when a human body directly touches the nozzle 16.

The micro gear pump 14 communicating with the nozzle 16 via the pipeline 17 functions as a feed apparatus for feeding the composition for forming a coating film accommodated in the container 15 to the nozzle 16. The micro gear pump 14 receives a feed of power supply from the low voltage power supply 11 and operates. Additionally, the micro gear pump 14 is configured in such a way as to feed a predetermined amount of the composition for forming a coating film to the nozzle 16 in response to the control by the auxiliary electric circuit 13.

The container 15 is connected to the micro gear pump 14 via a flexible pipeline 18. In the container 15, the composition for forming a coating film is accommodated. The container 15 is preferably an exchangeable cartridge type.

The electrostatic spray apparatus 10 having the above structure can be used as shown in, for example, Figure 2. Figure 2 shows a handy type electrostatic spray apparatus 10 having a size holdable by one hand. The electrostatic spray apparatus 10 shown in the same figure accommodates all the members of the structural diagram shown in Figure 1 inside a cylindrical case 20. The nozzle (not shown) is arranged at an end 10a of a longitudinal direction of the case 20. The nozzle is arranged in the case 20 with a coming-out direction of the composition in line with the longitudinal direction of the case 20 so that it protrudes toward the skin side. When the nozzle tip is arranged in such a way as to protrude toward the skin along with the longitudinal direction of the case 20, the composition for forming a coating film is less likely to attach to the case thereby stably forming a coating film.

When operating the electrostatic spray apparatus 10, a user, that is, a person, who forms a coating film on a site at which the coating film is formed on the skin by an electrostatic spray, holds the apparatus 10 with a hand and turns the end 10a of the apparatus 10 at which the nozzle (not shown) is arranged toward an application site to which an electrostatic spray is carried out.

Figure 2 shows a state in which the end 10a of the electrostatic spray apparatus 10 is turned toward the inner side of the user's forearm. Under this state, the apparatus 10 is switched ON to carry out the electrostatic spray method. When the apparatus 10 is turned on, an electric field is generated between the nozzle and the skin. In the embodiment shown in Figure 2, a positive high voltage is applied to the nozzle thereby making the skin a negative electrode. When the electric field is generated between the nozzle and the skin, the composition for forming a coating film at the nozzle tip section is polarized due to electrostatic induction and forms a corn shape at the tip part, and droplets of the charged composition for forming a coating film is discharged into the air from the corn tip toward the skin along with the electric field. As Component (b), a solvent, evaporates from the charged composition for forming a coating film discharged into the air, the composition for forming a coating film has an excessed charge density on the surface, proceeds in the air by Coulomb repulsion while repeatedly broken down, and reaches the skin. In this instance, when a viscosity of the composition for forming a coating film is suitably adjusted, the volatile substance, a solvent, is caused to volatilize from droplets while the composition is discharged into the air, and the polymer having a coating film forming ability, a solute, of Component (a) forms fibers as elongated by a potential difference while solidified, whereby the formed fibers are caused to deposit at an application site. For example, when a viscosity of the composition for forming a coating film is increased, the composition is likely to deposit at an application site in the form of fibers. Herewith, a porous coating film consisting of fibrous deposits is formed on the surface of an application site. Such a porous coating film consisting of fibrous deposits can also be formed by adjusting the distance between the nozzle and the skin or a voltage to be applied to the nozzle.

A high potential difference is being generated between the nozzle and the skin while carrying out the electrostatic spray method. However, the impedance is so significant that an electric current flowing through the human body is extremely small. For example, the present inventor confirmed that, for example, an electric current flowing through the human body while carrying out the electrostatic spray method is some digits smaller than an electric current flowing through the human body by static electricity generated in daily life.

When fibrous deposits are formed by the electrostatic spray method, a thickness of fibers when expressed by a diameter equivalent to a circle is preferably 10 nm or more, and more preferably 50 nm or more. Additionally, it is preferably 3,000 nm or less, more preferably 1,000 nm or less, and further preferably 800 nm or less. The thickness of a fiber can be measured by, for example, magnifying the fibers 10,000 times to observe using a scanning electron microscopic (SEM), excluding defects (fiber masses, fiber overlaps, and droplets) from the two-dimensional images, randomly selecting 10 fibers, drawing a line perpendicular to the longitudinal direction of the fibers, and directly reading a diameter of the fibers.

The fiber of the present invention is preferably a continuous fiber and preferably has a length of at least 100 times or more a thickness of the fiber. For examples, a formed coating film preferably comprises fibers comprising Component (a) and having a length of preferably 10 µm or more, more preferably 50 µm or more, and further preferably 100 µm or more.

In the present Description, a fiber having a length of 100 times or more a thickness of the fiber is defined as the "continuous fiber." The cross-sectional shape of the fiber is preferably circle or oval, and the fiber thickness refers to the diameter in the case of circle and a length of the major axis in the case of oval. Additionally, the coating film produced by the electrostatic spray method is preferably porous noncontinuous coating film consisting of one or more continuous fibrous deposits.

According to the electrostatic spray method, the composition for forming a coating film is charged and sprayed and affected by the moisture in the air when a humidity is high but can be less likely affected when a conductivity controlling agent is comprised.

A method for producing the composition for forming a coating film may stir a mixed solution containing all components but preferably has Step 1 for stirring Mixed solution 1 containing the components other than Component (a) and subsequently Step 2 for adding Component (a) followed by stirring and mixing. Alternatively, the method for producing the composition for forming a coating film may also have Step 1 for mixing Component (a) and a part of Component (b), Step 2 for mixing the remaining Component (b), Component (c) and Component (d), and Step 3 for mixing the mixture of Step 1 and the mixture of Step 2. These Step 1, Step 2, and Step 3 are preferably carried out at normal temperature of from 10°C to 30°C.

A distance between the nozzle and the skin is, depending on a voltage to be applied to the nozzle through, preferably 10 mm or more, more preferably 20 mm or more, and further preferably 40 mm or more. Additionally, it is preferably 160 mm or less, more preferably 140 mm or less, and further preferably 120 mm or less. A distance between the nozzle and the skin within this range can enhance the formability of a coating film. A distance between the nozzle and the skin can be measured by a commonly used non-contact sensor.

A basis weight of the coating film is, regardless of the coating film formed by the electrostatic spray method being porous or not, preferably 0.10 g/m² or more, and more preferably 1.0 g/m² or more. Additionally, it is preferably 50 g/m² or less, and more preferably 40 g/m² or less. A basis weight of the formed coating film is preferably 0.10 g/m² or more and 50 g/m² or less, and more preferably 1.0 g/m² or more and 40 g/m² or less. When a basis weight of the coating film is set as such, the skin compatibility, adhesion, scratch resistance, and sebum resistance of the coating film can be enhanced.

In reference with the embodiments described above, the present invention further discloses the following compositions and methods.

<1> A composition for forming a coating film, comprising the following Components (a), (b), (c), and (d):
   (a) a polymer having a coating film forming ability
   (b) one or more volatile substances selected from the group consisting of alcohols and ketones
   (c) a plasticizer
   (d) 3 mass% or more and 15 mass% or less of a non-volatile oil agent, other than Component (c), having a viscosity at 35°C of 50 mPa·s or more and 3,000 mPa·s or less.
<2> The composition for forming a coating film according to <1>, wherein Component (a) is a water-insoluble polymer having a coating film forming ability, and preferably one or more selected from the group consisting of completely-saponified polyvinyl alcohols insolubilizable after forming a coating film, partially-saponified polyvinyl alcohols crosslinkable after forming a coating film when used in combination with a crosslinking agent, oxazoline-modified silicones such as poly(N-propanoylethyleneimine)graft-dimethylsiloxane/y-aminopropylmethylsiloxane copolymer, polyvinyl acetal diethylamino acetates, zein (the principal protein of maize), polyesters, polylactic acids (PLA), acrylic resins such as polyacrylonitrile resin, and polymethacrylic acid resin, and polystyrene resins, polyvinyl butyral resins, polyethylene terephthalate resins, polybutylene terephthalate resins, polyurethane resins, polyamide resins, polyimide resins, and polyamideimide resins, and further preferably one or more selected from the group consisting of completely-saponified polyvinyl alcohols insolubilizable after forming a coating film, partially-saponified polyvinyl alcohols crosslinkable after forming a coating film when used in combination with a crosslinking agent, polyvinyl butyral resins, polyurethane resins, oxazoline-modified silicones, polyvinyl acetal diethylamino acetates, and zein.
<3> The composition for forming a coating film according to <1> or <2>, wherein a content of Component (a) is preferably 1.0 mass% or more, more preferably 2.0 mass% or more, further preferably 4.0 mass% or more, furthermore preferably 6.0 mass% or more, and furthermore preferably 8.0 mass% or more, and preferably 35 mass% or less, more preferably 30 mass% or less, further preferably 25 mass% or less, and furthermore preferably 20 mass% or less, and preferably 1.0 mass% or more and 30 mass% or less, more preferably 2.0 mass% or more and 25 mass% or less, further preferably 4.0 mass% or more and 20 mass% or less, and furthermore preferably 6.0 mass% or more and 20 mass% or less, and preferably 2.0 mass% or more and 35 mass% or less, more preferably 4.0 mass% or more and 30 mass% or less, further preferably 6.0 mass% or more and 30 mass% or less, furthermore preferably 6.0 mass% or more and 25 mass% or less, much preferably 8.0 mass% or more and 25 mass% or less, and much more preferably 8.0 mass% or more and 20 mass% or less.
<4> The composition for forming a coating film according to any of <1> to <3>, wherein a content of Component (a) is 2.0 mass% or more and 25 mass% or less.
<5> The composition for forming a coating film according to any of <1> to <3>, wherein a content of Component (a) is 6.0 mass% or more and 30 mass% or less.
<6> The composition for forming a coating film according to any of <1> to <5>, wherein Component (b) has a vapor pressure at 20°C of preferably 0.01 kPa or more and 106.66 kPa or less, more preferably 0.13 kPa or more and 66.66 kPa or less, further preferably 0.67 kPa or more and 40.00 kPa or less, furthermore preferably 1.33 kPa or more and 40.00 kPa or less, and much preferably 2.40 kPa or more and 40.00 kPa or less.
<7> The composition for forming a coating film according to any of <1> to <6>, wherein the alcohol of Component (b) is preferably one or more selected from the group consisting of monohydric chain fatty alcohols, monohydric cyclic fatty alcohols, and monohydric aromatic alcohols, preferably one or more selected from the group consisting of straight chain or branched chain monohydric chain fatty alcohols having 1 to 6 carbon atoms, monohydric cyclic fatty alcohols having 4 to 6 carbon atoms, benzyl alcohol, and phenyl ethyl alcohol, further preferably one or more selected from the group consisting of ethanol, isopropyl alcohol, n-butyl alcohol, phenyl ethyl alcohol, n-propanol, and n-pentanol.
<8> The composition for forming a coating film according to any of <1> to <7>, wherein the ketone of Component (b) is preferably a ketone having 2 alkyl groups having 1 to 4 carbon atoms, and further preferably one or more selected from the group consisting of acetones, methyl ethyl ketones, and methyl isobutyl ketones.
<9> The composition for forming a coating film according to any of <1> to <8>, wherein Component (b) is preferably one or more selected from the group consisting of ethanol, isopropyl alcohol, and n-butyl alcohol, more preferably one or more selected from the group consisting of ethanol and isopropyl alcohol, and further preferably ethanol.
<10> The composition for forming a coating film according to any of <1> to <9>, wherein a content of Component (b) is preferably 45 mass% or more, more preferably 50 mass% or more, further preferably 55 mass% or more, and furthermore preferably 60 mass% or more, and preferably 98.8 mass% or less, more preferably 98 mass% or less, further preferably 97 mass% or less, furthermore preferably 96 mass% or less, and much preferably 94 mass% or less, much more preferably 91 mass% or less, and much further preferably 88.5 mass% or less, and preferably 50 mass% or more and 98.8 mass% or less, more preferably 50 mass% or more and 98 mass% or less, further preferably 55 mass% or more and 96 mass% or less, and furthermore preferably 60 mass% or more and 94 mass% or less, and preferably 45 mass% or more and 97 mass% or less, more preferably 50 mass% or more and 94 mass% or less, further preferably 50 mass% or more and 91 mass% or less, and furthermore preferably 50 mass% or more and 88.5 mass% or less.
<11> The composition for forming a coating film according to any of <1> to <10>, wherein a content of Component (b) is 50 mass% or more and 98 mass% or less.
<12> The composition for forming a coating film according to any of <1> to <10>, wherein a content of Component (b) is 50 mass% or more and 91 mass% or less.
<13> The composition for forming a coating film according to any of <1> to <12>, wherein Component (c) is one or more selected from the group consisting of polyols, polyoxyalkylene glycols, polyoxyalkylene alkyl ethers, ester oils, silicone oils, hydrocarbon oils, liquid fat/oil, solid fat/oil, higher alcohols, and nonionic surfactants.
<14> The composition for forming a coating film according to any of <1> to <13>, wherein Component (c) is a compound easily interactable with the hydroxy group, the ester, and the acetal moiety in the structure of the polymer having a coating film forming ability, preferably one or more selected from the group consisting of polyols, polyoxyalkylene glycols, polyoxyalkylene alkyl ethers, specific ester oils, specific silicone oils, and nonionic surfactants, and more preferably one or more selected from the group consisting of polyols, polyoxyalkylene glycols, glycerin monofatty acid esters, glycerin difatty acid esters, malic acid diesters, N-acyl amino acid esters, ethylhexyl methoxycinnamate, and alkyl benzoates.
<15> The composition for forming a coating film according to any of <1> to <14>, wherein a content of Component (c) is preferably 0.10 mass% or more, more preferably 0.50 mass% or more, further preferably 1.0 mass% or more, and furthermore preferably 1.5 mass% or more, and preferably 30 mass% or less, more preferably 25 mass% or less, further preferably 20 mass% or less, and furthermore preferably 15 mass% or less, and preferably 0.10 mass% or more and 30 mass% or less, more preferably 0.50 mass% or more and 25 mass% or less, further preferably 1.0 mass% or more and 20 mass% or less, and furthermore preferably 1.0 mass% or more and 15 mass% or less, and preferably 0.50 mass% or more and 30 mass% or less, more preferably 1.0 mass% or more and 25 mass% or less, further preferably 1.0 mass% or more and 20 mass% or less, and furthermore preferably 1.5 mass% or more and 15 mass% or less.
<16> The composition for forming a coating film according to any of <1> to <15>, wherein a content of Component (c) is 0.50 mass% or more and 25 mass% or less.
<17> The composition for forming a coating film according to any of <1> to <15>, wherein a content of Component (c) is 1.0 mass% or more and 20 mass% or less.
<18> The composition for forming a coating film according to any of <1> to <17>, wherein a viscosity of Component (d) at 35°C is preferably 100 mPa·s or more and 2,800 mPa·s or less, more preferably 150 mPa·s or more and 2,500 mPa·s or less, and further preferably 200 mPa·s or more and 2,000 mPa·s or less.
<19> The composition for forming a coating film according to any of <1> to <18>, wherein Component (d) is one or more selected from the group consisting of ester oils and hydrocarbon oils.
<20> The composition for forming a coating film according to any of <1> to <19>, wherein Component (d) is one or more non-volatile oil agents selected from the group consisting of glycerides such as pentaerythrityl tetraethylhexanoate, glyceryl diisostearate, triethylhexanoin, pentaerythrityl tetra(behenate/benzoate/ethylhexanoate), dipentaerythritol fatty acid ester, glycerin di-2-heptylundecanoate, glycerin trioctanoate, glycerin triisopalmitate, glycerin tri-2-ethylhexanoate, glycerin trimyristate, glyceride tri-2-heptyl undecanoate, acetoglyceride, glycerin monofatty acid ester, glycerin difatty acid ester, and glycerin trifatty acid ester, acyl amino acid diesters such as di(phytosteryl/octyldodecyl) lauroyl glutamate, neopentyl glycol dicaprate, liquid paraffins, isododecane, paraffin waxes, and vaselines.
<21> The composition for forming a coating film according to any of <1> to <20>, wherein a content of Component (d) is preferably 3 mass% or more and 15 mass% or less, more preferably 3.5 mass% or more and 15 mass% or less, further preferably 4 mass% or more and 15 mass% or less, and furthermore preferably 5 mass% or more and 15 mass% or less.
<22> The composition for forming a coating film according to any of <1> to <21>, wherein a content mass ratio of Component (a) to Component (c), ((a)/(c)), is preferably 0.033 or more, more preferably 0.10 or more, further preferably 0.20 or more, furthermore preferably 0.40 or more, much preferably 0.80 or more, much more preferably 1.0 or more, and much further preferably 1.5 or more, and preferably 300 or less, more preferably 60 or less, further preferably 30 or less, furthermore preferably 20 or less, much preferably 15 or less, much more preferably 10 or less, and much further preferably 8.0 or less, and preferably 0.033 or more and 300 or less, more preferably 0.10 or more and 60 or less, further preferably 0.20 or more and 30 or less, furthermore preferably 0.40 or more and 10 or less, and much preferably 1.0 or more and 10 or less, and preferably 0.80 or more and 20 or less, more preferably 1.0 or more and 15 or less, further preferably 1.0 or more and 10 or less, and furthermore preferably 1.5 or more and 8.0 or less.
<23> The composition for forming a coating film according to any of <1> to <22>, wherein a content mass ratio of Component (a) to Component (c), ((a)/(c)), is 0.2 or more and 30 or less.
<24> The composition for forming a coating film according to any of <1> to <22>, wherein a content mass ratio of Component (a) to Component (c), ((a)/(c)), is 1.0 or more and 10 or less.
<25> The composition for forming a coating film according to any of <1> to <24>, wherein a content mass ratio of Component (c) to Component (d), ((c)/(d)), is preferably 0.0033 or more, more preferably 0.0050 or more, further preferably 0.0066 or more, furthermore preferably 0.01 or more, much preferably 0.03 or more, much more preferably 0.05 or more, much further preferably 0.066 or more, much furthermore preferably 0.1 or more, and much furthermore preferably 0.2 or more, and preferably 30 or less, more preferably 25 or less, further preferably 20 or less, furthermore preferably 15 or less, much preferably 10 or less, much more preferably 7.0 or less, much further preferably 5 or less, and much furthermore preferably 3 or less, and preferably 0.0033 or more and 30 or less, more preferably 0.0050 or more and 25 or less, further preferably 0.0066 or more and 20 or less, furthermore preferably 0.01 or more and 15 or less, preferably 0.03 or more and 10 or less, more preferably 0.05 or more and 7.0 or less, further preferably 0.066 or more and 5 or less, furthermore preferably 0.10 or more and 5 or less, and much furthermore preferably 0.2 or more and 3 or less.
<26> The composition for forming a coating film according to any of <1> to <25>, wherein a content mass ratio of Component (c) to Component (d), ((c)/(d)), is 0.03 or more and 10 or less.
<27> The composition for forming a coating film according to any of <1> to <25>, wherein a content mass ratio of Component (c) to Component (d), ((c)/(d)), is 0.2 or more and 3 or less.
<28> The composition for forming a coating film according to any of <1> to <27>, wherein a content mass ratio of Component (a) to Component (b), ((a)/(b)), is preferably 0.010 or more, more preferably 0.060 or more, further preferably 0.080 or more, furthermore preferably 0.10 or more, much preferably 0.11 or more, and much more preferably 0.12 or more, and preferably 0.60 or less, more preferably 0.45 or less, further preferably 0.35 or less, furthermore preferably 0.33 or less, much preferably 0.30 or less, much more preferably 0.25 or less, much further preferably 0.20 or less, and much further preferably 0.18 or less, and preferably 0.010 or more and 0.60 or less, more preferably 0.060 or more and 0.33 or less, further preferably 0.10 or more and 0.25 or less, furthermore preferably 0.11 or more and 0.20 or less, and much preferably 0.12 or more and 0.18 or less, and preferably 0.060 or more and 0.45 or less, more preferably 0.080 or more and 0.35 or less, further preferably 0.10 or more and 0.33 or less, furthermore preferably 0.11 or more and 0.30 or less, and much preferably 0.12 or more and 0.25 or less.
<29> The composition for forming a coating film according to any of <1> to <28>, wherein a content mass ratio of Component (a) to Component (b), ((a)/(b)), is 0.10 or more and 0.25 or less.
<30> The composition for forming a coating film according to any of <1> to <28>, wherein a content mass ratio of Component (a) to Component (b), ((a)/(b)), is 0.11 or more and 0.30 or less.
<31> The composition for forming a coating film according to any of <1> to <30>, wherein a content mass ratio of Component (a) to Component (d), ((a)/(d)), is preferably 0.0033 or more, more preferably 0.005 or more, further preferably 0.0066 or more, furthermore preferably 0.01 or more, much preferably 0.02 or more, much more preferably 0.03 or more, much more preferably 0.066 or more, much more preferably 0.1 or more, much more preferably 0.3 or more, much more preferably 0.5 or more, and much more preferably 0.8 or more, and preferably 30 or less, more preferably 25 or less, further preferably 20 or less, furthermore preferably 15 or less, much preferably 13 or less, much more preferably 10 or less, and much further preferably 8.0 or less, and preferably 0.0033 or more and 30 or less, more preferably 0.005 or more and 25 or less, further preferably 0.0066 or more and 20 or less, furthermore preferably 0.01 or more and 15 or less, much preferably 0.02 or more and 13 or less, preferably 0.03 or more and 10 or less, more preferably 0.066 or more and 10 or less, further preferably 0.10 or more and 8.0 or less, furthermore preferably 0.30 or more and 8.0 or less, furthermore preferably 0.50 or more and 8.0 or less, and furthermore preferably 0.80 or more and 8.0 or less.
<32> The composition for forming a coating film according to any of <1> to <31>, wherein a content mass ratio of Component (a) to Component (d), ((a)/(d)), is 0.02 or more and 13 or less.
<33> The composition for forming a coating film according to any of <1> to <31>, wherein a content mass ratio of Component (a) to Component (d), ((a)/(d)), is 0.80 or more and 8.0 or less.
<34> The composition for forming a coating film according to any of <1> to <33> comprising components selected from the group consisting of conductivity controlling agents, oil agents other than Components (c) and (d), coloring pigments, extender pigments, dyes, fragrances, repellents, antioxidants, stabilizers, preservatives, vitamins and water.
<35> The composition for forming a coating film according to <34>, wherein the conductivity controlling agent is preferably a component achieving a conductivity of the composition for forming a coating film at 25°C of 10 µS/cm or more and 300 µS/cm or less.
<36> The composition for forming a coating film according to <34> or <35>, wherein the conductivity controlling agent is preferably an alkali metal salt or an ammonium salt, more preferably an ionic surfactant, and further preferably one or more selected from the group consisting of cationic surfactants and anionic surfactants.
<37> The composition for forming a coating film according to any of <34> to <36>, wherein the conductivity controlling agent is one or more selected from the group consisting of quaternary ammonium salts and acyl amino acid salts.
<38> The composition for forming a coating film according to any of <34> to <37>, wherein a content of the conductivity controlling agent is preferably 0.010 mass% or more, more preferably 0.050 mass% or more, and further preferably 0.10 mass% or more, and preferably 10 mass% or less, more preferably 8.0 mass% or less, further preferably 6.0 mass% or less, furthermore preferably 2.5 mass% or less, and much preferably 2.0 mass% or less, and preferably 0.010 mass% or more and 10 mass% or less, more preferably 0.050 mass% or more and 8.0 mass% or less, further preferably 0.10 mass% or more and 6.0 mass% or less, furthermore preferably 0.10 mass% or more and 2.5 mass% or less, and much preferably 0.10 mass% or more and 2.0 mass% or less.
<39> The composition for forming a coating film according to any of <1> to <38>, wherein a viscosity of the composition for forming a coating film at 25°C is preferably 2 mPa·s or more, more preferably 5 mPa·s or more, further preferably 10 mPa·s or more, furthermore preferably 30 mPa·s or more, much preferably 50 mPa·s or more, and much more preferably 80 mPa·s or more, and preferably 3,000 mPa·s or less, more preferably 2,000 mPa·s or less, further preferably 1,500 mPa·s or less, furthermore preferably 1,000 mPa·s or less, much preferably 800 mPa·s or less, and much more preferably 500 mPa·s or less, and preferably 2 mPa·s or more and 3,000 mPa·s or less, more preferably 5 mPa·s or more and 2,000 mPa·s or less, further preferably 10 mPa·s or more and 1,500 mPa·s or less, furthermore preferably 30 mPa·s or more and 1,000 mPa·s or less, much preferably 50 mPa·s or more and 800 mPa·s or less, and much more preferably 80 mPa·s or more and 500 mPa·s or less.
<40> The composition for forming a coating film according to any of <1> to <39>, which is a composition for forming a coating film formed of fibers directly on the skin by an electrostatic spray.
<41> A sheet-like composition for skin obtained by electrostatically spraying a composition comprising the following Components (a), (b), (c), and (d):
   (a) a polymer having a coating film forming ability
   (b) one or more volatile substances selected from the group consisting of alcohols and ketones
   (c) a plasticizer
   (d) 3 mass% or more and 15 mass% or less of a non-volatile oil agent, other than Component (c), having a viscosity at 35°C of 50 mPa·s or more and 3,000 mPa·s or less.
<42> The sheet-like composition for skin according to <41>, wherein Component (d) is one or more non-volatile oil agents selected from the group consisting of pentaerythrityl tetraethylhexanoate, glyceryl diisostearate, triethylhexanoin, pentaerythrityl tetra(behenate/benzoate/ethylhexanoate), dipentaerythritol fatty acid esters, glycerin di-2-heptylundecanoate, glycerin trioctanoate, glycerin triisopalmitate, glycerin tri-2-ethylhexanoate, glycerin trimyristate, glyceride tri-2-heptyl undecanoate, acetoglycerides, glycerin monofatty acid esters, glycerin difatty acid esters, glycerin trifatty acid esters, di(phytosteryl/octyldodecyl) lauroyl glutamate, neopentyl glycol dicaprate, liquid paraffins, isododecane, paraffin waxes, and vaselines.
<43> The sheet-like composition for skin according to <41> or <42>, wherein Component (c) plasticizer is one or more selected from the group consisting of polyols, polyoxyalkylene glycols, glycerin monofatty acid esters, glycerin difatty acid esters, malic acid diesters, N-acyl amino acid esters, ethylhexyl methoxycinnamate, and alkyl benzoates.

### Example

Hereinafter, the present invention is described in further detail in reference to Examples. However, the scope of the present invention is not limited to these Examples. "%" means "mass%" unless otherwise specified.

### [Test Example 1]

### [Examples 1-7, Comparative Examples 1-4]

### (1) Preparation of the composition for forming a coating film

Polyvinyl butyral (manufactured by Sekisui Chemical Co., Ltd.: tradename; S-LEC B BM-1) was used as Component (a) and synthetic alcohol, 99 degrees (manufactured by Japan Synthetic Alcohol Co., Ltd.) was used as Component (b) for the composition for forming a coating film. Additionally, polyethylene glycol (average molecular weight 400) was used as Component (c), and as Component (d), (d1) neopentyl glycol dicaprate and (d2) dipentaerythrit fatty acid ester (melting point 50°C) were mixed to adjust the viscosity at 35°C and used. The content of each component shown in Tables 1 and 2 is an effective amount and the unit is mass%.

A mixture of Component (a) and Component (b) mixed homogeneously in advance, and Component (c) and Component (d) were each weighed and added to a 1,000 mL-SUS304 measuring cup (manufactured by TRUSCO NAKAYAMA CORPORATION) and mixed by stirring for 10 minutes at room temperature (from 20°C to 30°C) using a homogenizing disperser (manufactured by PRIMIX Corporation: T.K. Robomix). For this operation, the blade diameter of the disperser used was 50 mm, and a rotation speed was 3,000 rpm. After mixing by stirring, a homogeneous clear solution was obtained. This was considered as the composition for forming a coating film. The preparative scale of the composition for forming a coating film was 500 g in terms of the total weight of the composition for forming a coating film.

### (2) Viscosity of Component (d)

The viscosity of Compound (d), after mixing two types of oil agents, stored in a water bath for 24 hours at 35°C and measured at a measurement temperature of 35°C using a B type viscometer. The measurement temperature herein refers to a temperature of Component (d). The measurement was carried out using a B type viscometer (TVB-10M) manufactured by TOKI SANGYO CO., LTD. as the B type viscometer, with the rotor type of M1, M2, M4, at a rotation speed of from 3.0 rpm to 60 rpm.

### (3) Electrostatic spray step

Using the electrostatic spray apparatus 10 having the structure shown in Figure 1 and the appearance shown in Figure 2, the composition for forming a coating film was electrostatically sprayed for 30 seconds to a hole part (frame part) cut out in 3 x 8 cm from a 5 x 10 cm aluminum foil to form a coating film formed of fibers. The polymer amount was 6.6 mg (0.275 mg/cm²).

### (4) Evaluation on sebum resistance

Ten µL of oleic acid (manufactured by FUJIFILM Wako Pure Chemical Corporation: Wako 1st Grade), as a sebum model, was dropped onto the coating film formed of fibers formed within the frame on the aluminum foil, and the time for the coating film to have a hole was measured.

Results are shown in Table 1 and Table 2.

**[Table 1]**

| | | | Comparative Example 1 | Example 1 | Example 2 | Example 3 | Example 4 | Comparative Example 2 | Comparative Example3 |
|---|---|---|---|---|---|---|---|---|---|
| (a) Polyvinyl butyral | | % | 12.00 | 12.00 | 12.00 | 12.00 | 12.00 | 12.00 | 12.00 |
| (c) PEG-400 | | % | 4.00 | 4.00 | 4.00 | 4.00 | 4.00 | 4.00 | 4.00 |
| Volatile silicone oil | | % | 7.00 | 7.00 | 7.00 | 7.00 | 7.00 | 7.00 | 7.00 |
| (d) Non-volatile oil agent | (d1) Neopentyl glycol dicaprate | % | 7.00 | 5.25 | 4.20 | 3.50 | 2.80 | 1.75 | 0.00 |
| | (d2) Dipentaerythrit fatty acid ester | % | 0.00 | 1.75 | 2.80 | 3.50 | 4.20 | 5.25 | 7.00 |
| (b) Ethanol | | % | 70.00 | 70.00 | 70.00 | 70.00 | 70.00 | 70.00 | 70.00 |
| | | | | | | | | | |
| (d) Viscosity of non-volatile oil agent (@35°C) | | mPa·s | 11.3 | 83.2 | 201 | 493 | 1500 | 6520 | 123100 |
| | | | | | | | | | |
| Time for a coating film to have a hole (sebum resistance) | | min. | 50 | 150 | >270 | >270 | >270 | 30 | 3 |

**[Table 2]**

| | | | Example 3 | Comparative Example 4 | Example 5 | Example 6 | Example 7 |
|---|---|---|---|---|---|---|---|
| (a) Polyvinyl butyral | | % | 12.00 | 12.00 | 12.00 | 12.00 | 12.00 |
| (c) PEG-400 | | % | 4.00 | 4.00 | 4.00 | 4.00 | 4.00 |
| Volatile silicone oil | | % | 7.00 | 7.00 | 7.00 | 7.00 | 7.00 |
| (d) Non-volatile oil agent | (d1) Neopentyl glycol dicaprate | % | 3.50 | 1.00 | 2.00 | 5.00 | 6.00 |
| | (d2) Dipentaerythrit fatty acid ester | % | 3.50 | 1.00 | 2.00 | 5.00 | 6.00 |
| (b) Ethanol | | % | 70.00 | 75.00 | 73.00 | 67.00 | 65.00 |
| | | | | | | | |
| (d) Viscosity of non-volatile oil agent (@35°C) | | mPa·s | 493 | 493 | 493 | 493 | 493 |
| | | | | | | | |
| Time for a coating film to have a hole (sebum resistance) | | min. | >270 | 30 | 120 | >270 | >270 |

(a) Polyvinyl butyral (manufactured by Sekisui Chemical Co., Ltd.: tradename; S-LEC B BM-1)
(b) Ethanol (manufactured by Japan Synthetic Alcohol Co., Ltd.: synthetic alcohol, 99 degrees)
(c) Polyethylene glycol (manufactured by SANYO CHEMICAL INDUSTRIES, LTD.: PEG-400(-G))
(d1) Neopentyl glycol dicaprate (manufactured by The Nisshin OilliO Group, Ltd.: ESTEMOL N-01)
(d2) Dipentaerythrit fatty acid ester (manufactured by The Nisshin OilliO Group, Ltd.: COSMOL 168EV)
Volatile silicone oil (manufactured by Shin-Etsu Chemical Co., Ltd.: KF-96L-2CS(-G))

Table 1 reveals that sebum resistance is excellent when the viscosities of Component (d) at 35°C are 50 mPa·s or more and 3,000 mPa·s or less. Additionally, Table 2 reveals that sebum resistance is excellent when content of Component (d) is 3 mass% or more and 15 mass% or less.

As a result of electrostatically spraying the compositions for forming a coating film of the examples in Table 1 and Table 2 to form a coating film formed of fibers directly on the skin and evaluating on the adhesion and scratch resistance of these coating films, All the coating films had excellent adhesion and scratch resistance.

### [Test Example 2]

### [Examples 8 to 11]

### (1) Preparation of the composition for forming a coating film

Polyvinyl butyral was used as Component (a) and synthetic alcohol, 99 degrees (manufactured by Japan Synthetic Alcohol Co., Ltd.) was used as Component (b) for the composition for forming a coating film. Additionally, polyethylene glycol (average molecular weight 400), glycerin, PEG/PPG/polybutylene glycol-8/5/3 glycerin, 1,3-butylene glycol, or ethylhexyl methoxycinnamate was used as Component (c), and as Component (d), neopentyl glycol dicaprate, dipentaerythrit fatty acid ester (melting point 50°C), isononyl isononanoate, or isododecane was mixed with dextrin palmitate to adjust the viscosity at 35°C and used. The content of each component shown in Table 3 is an effective amount and the unit is mass%.

A mixture of Component (a) and Component (b) mixed homogeneously in advance, and Component (c) and Component (d) were each weighed and added to a 1,000 mL-SUS304 measuring cup (manufactured by TRUSCO NAKAYAMA CORPORATION) and mixed by stirring for 10 minutes at room temperature (from 20°C to 30°C) using a homogenizing disperser (manufactured by PRIMIX Corporation: T.K. Robomix). For this operation, the blade diameter of the disperser used was 50 mm, and a rotation speed was 3,000 rpm. After mixing by stirring, a homogeneous clear solution was obtained. This was considered as the composition for forming a coating film. The preparative scale of the composition for forming a coating film was 500 g in terms of the total weight of the composition for forming a coating film.

### (2) Viscosity of Component (d)

The viscosity of Compound (d), after mixing non-volatile oil agent and dextrin palmitate, stored in a water bath for 24 hours at 35°C and measured at a measurement temperature of 35°C using a B type viscometer. The measurement temperature herein refers to a temperature of Component (d). The measurement was carried out using a B type viscometer (TVB-10M) manufactured by TOKI SANGYO CO., LTD. as the B type viscometer, with the rotor types of M1, M2, and M4 at a rotation speed of from 3.0 rpm to 60 rpm.

### (3) Electrostatic spray step

Using the electrostatic spray apparatus 10 having the structure shown in Figure 1 and the appearance shown in Figure 2, the composition for forming a coating film was electrostatically sprayed for 30 seconds to a hole part (frame part) cut out in 3 x 8 cm from a 5 x 10 cm aluminum foil to form a coating film formed of fibers. The polymer amount was 6.6 mg (0.275 mg/cm²).

### (4) Evaluation on sebum resistance

Ten µL of oleic acid (manufactured by FUJIFILM Wako Pure Cemical Corporation: Wako 1st Grade), as a sebum model, was dropped onto the coating film formed of fibers formed within the frame on the aluminum foil, and the time for the coating film to have a hole was measured.

Results are shown in Table 3.

**[Table 3]**

| | | | Example 8 | Example 9 | Example 10 | Example 11 |
|---|---|---|---|---|---|---|
| (a) | Polyvinyl butyral | % | 11.0 | 11.0 | 11.0 | 11.0 |
| (c) | PEG-400 *1 | % | 4.0 | - | - | - |
| | Glycerin | % | 1.0 | - | - | - |
| | PEG/PPG/polybutylene glycol-8/5/3 glycerin *2 | % | - | 1.0 | - | - |
| | 1 ,3-Buthylene glycol | % | - | - | 1.0 | - |
| | Ethylhexyl methoxycinnamate | % | - | - | - | 2.0 |
| | Volatile silicone oil *3 | % | 7.0 | 7.0 | 7.0 | 7.0 |
| (d) | Neopentyl glycol dicaprate *4 | % | 7.0 | - | - | 6.0 |
| | Dipentaerythrit fatty acid ester *5 | % | 5.0 | - | - | 4.0 |
| | Isononyl isononanoate | % | - | 10.0 | - | - |
| | Isododecane *6 | % | - | - | 10.0 | - |
| | Dextrin palmitate | % | - | 0.5 | 0.7 | - |
| (b) | Ethanol *7 | % | 65.0 | 70.5 | 70.3 | 70.0 |
| | | | | | | |
| (d) Viscosity of non-volatile oil agent (@35°C) | | mPa·s | 201 | 594 | 354 | 201 |
| | | | | | | |
| Time for a coating film to have a hole (sebum resistance) | | min. | >270 | >270 | >270 | >270 |

*1: PEG-400(-G) (manufactured by SANYO CHEMICAL INDUSTRIES, LTD.)
*2: Wilbride S-753 (NOF CORPORATION)
*3: KF-96L-2CS(-G) (manufactured by Shin-Etsu Chemical Co., Ltd.)
*4: ESTEMOL N-01 (manufactured by The Nisshin OilliO Group, Ltd.)
*5: COSMOL 168EV (manufactured by The Nisshin OilliO Group, Ltd.)
*6: Marukazole R (manufactured by MARUZEN PETROCHEMICAL CO., LTD.)
*7: Synthetic alcohol, 99 degrees (manufactured by Japan Synthetic Alcohol Co., Ltd.)

Table 3 reveals that the sebum resistance was excellent in all examples.

### Reference Signs List

- 10: Electrostatic spray apparatus
- 11: Low voltage power supply
- 12: High voltage power supply
- 13: Auxiliary electric circuit
- 14: Micro gear pump
- 15: Container
- 16: Nozzle
- 17: Pipeline
- 18: Flexible pipeline
- 19: Electric current limiting resistor
- 20: Case

## Claims

1. A composition for forming a coating film, comprising the following Components (a), (b), (c), and (d):
(a) a polymer having a coating film forming ability
(b) one or more volatile substances selected from the group consisting of alcohols and ketones
(c) a plasticizer
(d) 3 mass% or more and 15 mass% or less of a non-volatile oil agent, other than Component (c), having a viscosity at 35°C of 50 mPa·s or more and 3,000 mPa·s or less.

2. The composition for forming a coating film according to claim 1, wherein Component (d) is an oil component comprising one or more non-volatile oil agents selected from the group consisting of ester oils, silicone oils, hydrocarbon oils, liquid fat/oil, solid fat/oil, and higher alcohols, other than Component (c).

3. The composition for forming a coating film according to claim 1 or 2, wherein a viscosity of Component (d) at 35°C is 100 mPa·s or more and 3,000 mPa·s or less.

4. The composition for forming a coating film according to any one of claims 1 to 3, wherein a content of Component (d) is 3.5 mass% or more and 15 mass% or less.

5. The composition for forming a coating film according to any one of claims 1 to 4, wherein a content mass ratio of Component (c) to Component (d), ((c)/(d)), is 0.03 or more and 10 or less.

6. The composition for forming a coating film according to any one of claims 1 to 5, wherein a content mass ratio of Component (c) to Component (d), ((c)/(d)), is 0.2 or more and 3 or less.

7. The composition for forming a coating film according to any one of claims 1 to 6, wherein a content mass ratio of Component (a) to Component (d), ((a)/(d)), is 0.02 or more and 13 or less.

8. The composition for forming a coating film according to any one of claims 1 to 7, wherein a content mass ratio of Component (a) to Component (d), ((a)/(d)), is 0.8 or more and 8.0 or less.

9. The composition for forming a coating film according to any one of claims 1 to 8, wherein Component (d) is one or more non-volatile oil agents selected from the group consisting of pentaerythrityl tetraethylhexanoate, glyceryl diisostearate, triethylhexanoin, pentaerythrityl tetra(behenate/benzoate/ethylhexanoate), dipentaerythritol fatty acid esters, glycerin di-2-heptylundecanoate, glycerin trioctanoate, glycerin triisopalmitate, glycerin tri-2-ethylhexanoate, glycerin trimyristate, glyceride tri-2-heptyl undecanoate, acetoglycerides, glycerin monofatty acid esters, glycerin difatty acid esters, glycerin trifatty acid esters, di(phytosteryl/octyldodecyl) lauroyl glutamate, neopentyl glycol dicaprate, liquid paraffins, isododecane, paraffin waxes, and vaselines.

10. The composition for forming a coating film according to any one of claims 1 to 9, wherein Component (c) plasticizer is one or more selected from the group consisting of polyols, polyoxyalkylene glycols, glycerin monofatty acid esters, glycerin difatty acid esters, malic acid diesters, N-acyl amino acid esters, ethylhexyl methoxycinnamate, and alkyl benzoates.

11. The composition for forming a coating film according to any one of claims 1 to 10, which is a composition for forming a coating film formed of fibers directly on the skin by an electrostatic spray.

12. A sheet-like composition for skin obtained by electrostatically spraying a composition comprising the following Components (a), (b), (c), and (d):
(a) a polymer having a coating film forming ability
(b) one or more volatile substances selected from the group consisting of alcohols and ketones
(c) a plasticizer
(d) 3 mass% or more and 15 mass% or less of a non-volatile oil agent, other than Component (c), having a viscosity at 35°C of 50 mPa·s or more and 3,000 mPa·s or less.

13. The sheet-like composition for skin according to claim 12, wherein Component (d) is one or more non-volatile oil agents selected from the group consisting of pentaerythrityl tetraethylhexanoate, glyceryl diisostearate, triethylhexanoin, pentaerythrityl tetra(behenate/benzoate/ethylhexanoate), dipentaerythritol fatty acid esters, glycerin di-2-heptylundecanoate, glycerin trioctanoate, glycerin triisopalmitate, glycerin tri-2-ethylhexanoate, glycerin trimyristate, glyceride tri-2-heptyl undecanoate, acetoglycerides, glycerin monofatty acid esters, glycerin difatty acid esters, glycerin trifatty acid esters, di(phytosteryl/octyldodecyl) lauroyl glutamate, neopentyl glycol dicaprate, liquid paraffins, isododecane, paraffin waxes, and vaselines.

14. The sheet-like composition for skin according to claim 12 or 13, wherein Component (c) plasticizer is one or more selected from the group consisting of polyols, polyoxyalkylene glycols, glycerin monofatty acid esters, glycerin difatty acid esters, malic acid diesters, N-acyl amino acid esters, ethylhexyl methoxycinnamate, and alkyl benzoates.
